# EUROPEAN PATENT SPECIFICATION

(11) **EP 0 372 277 B1**
(45) Date of publication and mention of the grant of the patent: **06.07.1994**
(21) Application number: 89121334.0
(22) Date of filing: 17.11.1989
(51) Int. Cl.: A61M 5/14, A61M 39/00

(54) **Prefilled syringe delivery system**
Verabreichungssystem für vorgefüllte Spritze
Système d'administration de seringue préremplie

(30) Priority: 06.12.1988 US 280368
(43) Date of publication of application: 13.06.1990
(73) Proprietor: BAXTER INTERNATIONAL INC., Deerfield, IL 60015 (US); ASTRA PHARMACEUTICAL PRODUCTS, INC., Westboro Massachusetts (US)
(72) Inventor: Kern, Steven E., Sommerville MA (US); Lombardi, William V., Northboro MA (US); Skakoon, James G., Melrose MA (US)
(74) Representative: Schroeter, Helmut

(56) References cited:
- WO-A-87/07159
- CH-A- 400 005
- US-A- 3 986 508
- US-A- 4 232 669
- US-A- 4 511 359

## Description

### Field of the Invention

The present invention relates, in general, to an integrated delivery system using a prefilled syringe. In particular, the present invention relates to the use of a permanently protected needle in such a system, together with guiding means for the needle, which prevent tampering and disconnection of the system and which cooperate with a syringe pump.

### Description of the Prior Art

In the art of medical syringes, it is well known to provide a protected needle which is selectively exposed for a limited period for injection before being returned to a protected state. This protected state of the needle allows the syringe to be easily handled by medical pesonnel wish reduced risk of accidental injury, injection and subsequent infection from an exposed needle. However, such syringes are still hazadous in that they may be mishandled by medical personnel so that a needle is inadvertently exposed leading to the possibility of such accidental injury, injection and infection.

Examples of these syringes are shown in U. S. Patent Nos. 4,693,708 and 4,681,567. The 4,693,708 patent discloses a cylindrical housing of the needle guard which extends and retracts with respect to the syringe barrel. The 4,681,567 patent discloses a slidably mounted sheath which acts as a needle guard.

Moreover, it is well-known in the prior art to use a sealed hypodermic syringe. An example of such a syringe is found in U. S. Patent No. 3,825,003.

Further, in the prior art, it is well known to use a prefilled Syringe to deliver medicine or pharmaceutical product to separate medical apparatus for subsequent infusion into the patient. However, in order to be compatible with a wide range of apparatus, and possibly due to an ill-considered decision to borrow from the art of syringes which are used for direct injection into a patient (which fundamentally must provide an exposed needle to allow for direct injection), these prefilled syringes have typically included a needle which is either permanently exposed or selectively exposed.

This permanent or selective exposure of the needle leads to two disadvantages.

Firstly, the exposed needle is dangerous to medical personnel.

Secondly, the exposed needle typically has no means for guiding the needle into a shall insertion area of the aforementioned separate medical apparatus. Inaccurate insertion of the needle into the medical apparatus can result in contamination of the needle, damage to the needle or to the apparatus or injury to the user. A good example of such an apparatus is disclosed in U. S. Patent No. 4,232,669. This apparatus includes a needle which is protected by a protective sheath and a removable protective cap. In order to use this device, the cap is removed which exposes the needle beyond the protection of the detachable protective sheath.

None of these patents discloses the combination of a syringe with a tubing set, wherein a permanently shielded needle cooperates with guiding means for insertion of the needle into a puncture port of the tubing set, and wherein the guiding means forms a mechanical interference for a cooperation with the housing of a syringe pump to prevent tampering or disconnection of the whole device.

With an apparatus of this kind accurate guiding and positioning of the needle relative to the puncture port of the tubing set can be achieved. Thereby inadvertent dissociation of the assembly is avoided to protect the user from injury or to protect the patient from an interruption of the flow of the medical drug.

U.S. Patent No. 4.511.359 describes a connection device, which in a special embodiment (Fig. 8 A - C) shows a syringe with a needle, wherein the needle pierces and penetrates a diaphragm. The diaphragm is mounted in a connector member, which is maintained in alignment with the syringe by a tubular sheath. This sheath may be fixedly or slidably secured to either or both the syringe or the connector.

In this patent the tubular sheath must be slidably mounted, so that the needle is guided into the connector member for piercing the diaphragm. If the connector member is removed and the tubular sheath is slided into a backward position the needle is no longer shielded. Thus, the tubular sheath does not serve as a needle guard for a permanently shielded needle. No mention at all is made of a cooperation of the needle guiding means with a tubing set, which prevents tampering or disconnection of the whole device. This patent does not describe a cooperative interrelationship between a needle guard on a syringe and special guiding means to protect the user from inadvertent puncture by the needle and to accurately guide and position the needle relative to a puncture port in a tubing set. Further no cooperation of the guiding means with the housing of a syringe pump is described, to prevent inadvertent dissociation of the syringe and the tubing set.

In another special embodiment (Fig. 10 A and 10 B) the US Patent No. 4.511.359 describes a syringe with a needle with a resilience and slash or flexible tubular sheath. This sheath is attached to a barrel portion of the syringe and has an outersurface adaptable for fixedly attaching said sheath as by cement or the like. The syringe arranged in this embodiment is neither suitable to be engaged and disengaged with a syringe holder nor provided for a rotatable connection between holder and syringe giving the user the possibility to read the drug specification of the syringe printed on the outer periphery thereof.

The opening clause of new claim 1 relates to WO 87/07159 which describes a medical drug delivery system. A cartridge is mounted upon a receptacle, wherein the cartridge comprises a needle guard extending beyond the tip of the needle so that the needle is shielded, and wherein the needle guard serves as guiding means (Fig. 7). The receptacle is mounted upon a tubing set and comprises puncture port means, which have guiding means for the engagement with the needle guard, so that the needle is accurately guided and positioned to puncture the puncture port means when the cartridge is mounted upon the receptacle.

The cited application describes a general drug delivery system, but not the special realization with a syringe, as disclosed in the invention. More specifically in the prior art the needle guard is constructed such, that it rather serves as guiding means than as a needle guard. Because of the slot (120, Fig. 7) therein the needle is not completely shielded, so that there still exists a danger of injury for the user. Also no mention is made, that the needle guard is permanently attached to a cartridge holder or even integrally formed with it.

In the known delivery system two cannulas or needles are provided, which must be disposed in a particular position within the receptacle. This is provided for by the slot (120) in the needle guard into which the receptacle is inserted, and further by longitudinal channels (122), which fit over longitudinal key's (124) mounted about the puncture port means of the tubing set. In contrast to this realization the invention describes guiding means, in which the needle guard can be positioned around the puncture port means in any circumferential position. Therefore no channels, slots or keys are necessary in the guiding means.

According to feature i) of the new claim 1 the disc forms a mechanical interference with the housing of a syringe pump, cooperating to prevent tampering or disconnection. No mention of such features is made in the WO 87/07159.

It is therefore an object of the present invention to provide a prefilled syringe wherein the needle is permanently shielded.

It is therefore a further object of the present invention to provide a prefilled syringe which is constructed such as the user is protected from inadvertent puncture and injury by the needle and further includes a means for guiding the needle accurately into other medical apparatus.

As still another object of the present invention the guiding means is formed such, that it cooperates with the housing of a syringe pump to prevent tampering or disconnection of the whole device.

The object of WO 87/07159 is the passive delivery of a beneficial agent for a patient, wherein the agent is supplied by mixing a drug with a diluent. Thus, the object of this reference is completely different from the object of the invention. Also no hint is given in the cited reference or in a combination of the above mentioned references, how to solve the named objects of this invention or even a combination thereof.

### Summary of the Invention

The present invention is a prefilled syringe delivery system which includes a prefilled syringe with a needle permanently protected by a cylindrical guard which extends beyond the tip of the needle. The open end of the cylindrical guard serves as a guide for a puncture port to be accurately positioned with respect to the needle.

The puncture port leads to tubing which includes an anti-siphoning means and means for connection to other apparatus.

### Brief Description of the Drawings

Figure 1 discloses a side plan view of the prefilled syringe with the plunger at its withdrawn position, the piston screwably removed from the plunger, and a needle sheath over the needle.

Figure 2 discloses a side plan view of the prefilled syringe with the plunger at its withdrawn position, the piston screwably inserted into the plunger and the needle sheath removed.

Figure 3 discloses a cross-sectional view along section 3-3 of Figure 2.

Figure 4 discloses a side plan view of the prefilled syringe with the puncture port inserted over the needle.

Figure 5 discloses a side plan view of the tubing set of the apparatus, with a female liner connector shown in phantom under the puncture port.

### Detailed Description of the Preferred Embodiment

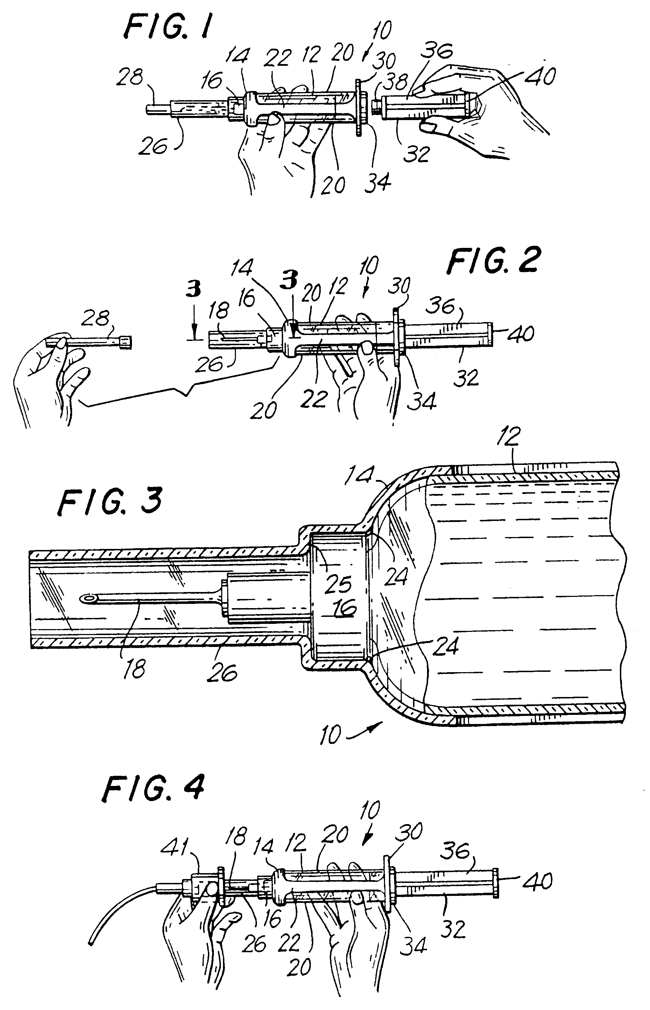

Referring now to the drawings in detail wherein like numerals refer to like elements throughout the several views, apparatus 10 is disclosed in Figures 1 and 2.

Syringe 12, which contains the pharmaceutical product, is held within syringe holder 14. Syringe 12 is preferably made of breakage resistant glass while syringe holder 14 is made of plastic or some similar material which is resistant to breakage. Needle hub 16 is attached to syringe 12 and, in turn, is attached to needle 18 (See Figure 3). The connections between syringe 12 and hub 16 and between hub 16 and needle 18 are, of course, liquid-tight.

The syringe holder 14 engages the outer curved cylindrical surface of syringe 12 with open portions 20 separated by a stem 22 in the intermediate portion thereof. This allows the user to grasp firmly either the syringe 12 or the syringe holder 14.

As shown more clearly in Figure 3, the syringe holder 14 further engages the needle hub 16 with a lip 24 as well as surface 25 which allow the syringe 12 and syringe holder 14 to be rotatable about one another while preventing any longitudinal movement of the syringe 12 with respect to the syringe holder 14.

Since each user of a medical drug delivery system before application is obligated to verify which type of medical drug the system contents there must be the possibility to read the drug specification of the syringe printed on the outer periphery thereof. In the case the user wants to read the printed specification of the syringe hidden by parts of the holder for example the stem 22, the user may rotate the syringe within the holder for that purpose.

Needle guard 26 is integrally formed on the proximate end of syringe holder 14 in the shape of a cylinder with the needle 18 as its longitudinal axis. The needle guard 26 extends from the proximate face of needle hub 16 to beyond the tip of a needle 18. Needle 18 is therefore permanently protected and not exposed as needle guard 26 is integral with syringe holder 14 and lip 24 working together with surface 25 prevents any relative longitudinal movement of syringe 12 and syringe holder 14.

Apparatus 10 further includes a needle sheath 28 which covers the needle 18 to provide further safety during transportation (See Figure 1) but is removed during use (See Figure 2).

The distal end of syringe holder 14 includes outwardly extending annular flanges 30 which serve as finger grips.

A plunger rod 32 is engaged through the distal end of the syringe 12. Piston 36 is comprised of a rubber plunger 34 and a plunger rod 32. Rubber plunger 34 engages the interior of syringe 12 is a liquid-tight but slidable manner. Plunger rod 32 includes a male threaded portion 38 on its proximate end which engages a female threaded portion (not shown) of rubber plunger 34 and a flat handle portion 40 on its distal end.

The female threaded portion of the rubber plunger 34 allows the plunger rod 32 to be disassembled during transportation, even if apparatus 10 is filled with pharmaceutical product, thereby resulting in a more compact package which is not as susceptible to inadvertent movement and loss of the pharmaceutical product.

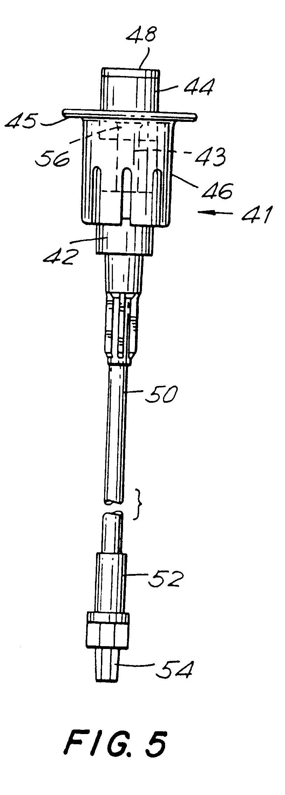

In order to use apparatus 10 effectively, tubing set 41 as disclosed in Figure 5 is supplied. Tubing set 41 includes an anti-siphoning check valve 42, such as is disclosed in U. S. Patent No. 4,535,820, and an input stem 43 leading to cylindrical input 44. Cylindrical input 44 has a diameter just less than the internal diameter of needle guard 26. During operation, check valve 42 has a positive crack pressure to help prevent uncontrolled syringe emptying. A tamper-resistant disk 46 surrounds anti-siphoning check valve 42 and includes an annular hub 45 which serves as a manual grip for tubing set 41. Disk 46 further forms a mechanical interference with the housing of a syringe pump (not shown) to help prevent tampering or inadvertent disconnection. Cylindrical input 44 includes a rubber puncture port 48 which serves as an injection site and further serves as a sterile connection prior to injection by needle 18. Low volume tubing 50 extends from check valve 42 to a lower luer connector 52 which is covered by a protective cap 54. In its preferred embodiment, tubing 50 is ninety-six inches long, resulting in a priming volume of 2.1 milliliters for the tubing set 41. Lower luer connector 52 provides a means for attachment to secondary medical devices (not shown) or to a direct injection needle (not shown) for direct infusion into the patient.

The cylindrical input 44 may be screwably removed from anti-siphoning check valve 42 to expose a female luer lock connector 56 on the end of stem 43 in order to adapt the tubing set 41 to a standard luer lock syringe (not shown).

In order to use apparatus 10, the user should firstly be skilled in aseptic technique and accepted IV (intravenous) practice. Syringe 12 is ordinarily provided prefilled with pharmaceutical product or medicine from the manufacturer or from a central location in the medical facility. The user screws plunger rod 32 into rubber plunger 34 so as to form a piston 36. The user removes needle guard 28. The user pushes the piston 36 to expel all air from the syringe 12. The user may wish to use antiseptic to sterilize puncture port 48. The user holds annular hub 45 of disk 46 and guides the cylindrical input 44 and puncture port 48 of tubing set 41 into the interior of needle guard 26. Further, as cylindrical input 44 and puncture port 48 are positioned in needle guard 26 prior to insertion of needle 18 through puncture port 48, needle 18 is accurately positioned against puncture port 48 prior to insertion. When needle 18 is inserted through puncture port 48, a liquid-proof engagement is formed between apparatus 10 and tubing set 41. The user removes protective cap 54 from lower luer connector 52 and lower luer connector is connected to either a direct injection needle (not shown) or to another medical device (not shown). The user pushes the piston 36 to expel all air from the syringe 12 and tubing set 41. The user inserts the syringe holder 14 and piston 36 into a medical pump (not shown) and makes the appropriate connection to the patient. The user then activates the medical pump.

In some rare applications not involving direct infusion into patients, the user may dispense with the medical pump and use piston 36 to manually inject the medicine or pharmaceutical product into another medical device (not shown).

Thus the several aforementioned objects and advantages are most effectively attained. Although a single preferred embodiment of the invention has been disclosed and described in detail herein, it should be understood that this invention is in no sense limited thereby and its scope is to be determined by that of the appended claims.

## Claims

1. A medical drug delivery system comprising:
a) a syringe (12) with a needle (18);
b) a piston (36) engaged by said syringe (12);
c) the needle having a tip extending from said syringe and including a passage for flow of liquid from said syringe through said needle in response to pushing said piston longitudinally through said syringe;
d) a needle guard (26) extending beyond the tip of the needle (18);
e) a tubing set (41) with a puncture port means (48), wherein the tubing of the tubing set (41) is extending from the puncture port means (48), and wherein the puncture port means (48) has a diameter equal to an interior diameter of the needle guard (26), so that when the puncture port means (48) is inserted into the needle guard (26) the needle (18) is accurately positioned against the puncture port means (48) and to thereby form a liquid-proof connection between the puncture port means (48) and the needle (18);
**characterized** in that
f) a syringe holder (14) is provided for holding the syringe (12);
g) the syringe holder (14) comprises a lip (24) and a surface (25) which engage with a needle hub (16) between the syringe (12) and the needle (18) so that the syringe (12) and the syringe holder (14) are rotatable about one another while longitudinal movement of the syringe (12) with respect to the syringe holder (14) is prevented;
h) the needle guard (26) is integrally formed with the syringe holder (14);
i) the syringe (12) is adapted to be automatically driven;
j) the tubing set (41) comprises a tamper-resistant disk (46) with an annular hub (45) serving as a manual grip, wherein the disk (46) forms a mechanical interference with the housing of a syringe pump, cooperating to prevent tampering or disconnection;
k) the puncture port means (48) is removable from the tubing set (41) in order to expose a female lock connector.

2. The system of claim 1, **characterized** in that
the tubing set (41) includes anti-siphoning means (42) between the puncture port means (48) and the tubing (50).

3. The system of claim 2, **characterized** in that
the puncture port means (48) is removable from the anti-siphoning means (42) in order to expose a female Luer lock connector (56).

4. The system of claim 3, **characterized** in that
the tubing (50) leads from the anti-siphoning means (42) to a male Luer lock connector (52).

5. The system of claim 1, **characterized** in that
the needle guard (26) is a hollow cylinder with the needle (18) extending along a portion of a longitudinal axis of the needle guard (26).

6. The system of claim 5, **characterized** in that
a needle sheath (28) is provided which removably fits over the needle (18).

## Patentansprüche

1. Verabreichungssystem für medizinische Arzneien mit
a) einer Spritze (12) mit einer Nadel (18);
b) einem Kolben (36), der von der Spritze (12) betätigt wird,
c) wobei die Nadel eine Spitze aufweist, die sich aus der Spritze erstreckt und einen Durchgang für einen Flüssigkeitsfluß von der Spritze durch die Nadel als Reaktion auf ein Drücken des Kolbens in Längsrichtung durch die Spritze aufweist;
d) einem Nadelschutz (26), der sich über die Spitze der Nadel (18) erstreckt;
e) mit einem Rohrset (41) mit einer Einstichstelleneinrichtung (48), wobei sich das Rohr des Rohrsets (41) von der Einstichstelleneinrichtung (48) erstreckt, und wobei die Einstichstelleneinrichtung (48) einen Durchmesser aufweist, der gleich einem inneren Durchmesser des Nadelschutzes (26) ist, so daß, wenn die Einstichstelleneinrichtung (48) in den Nadelschutz (26) eingesetzt ist, die Nadel (18) richtig gegen die Einstichstelleneinrichtung (48) positioniert ist, und um dadurch eine flüssigkeitsdichte Verbindung zwischen der Einstichstelleneinrichtung (48) und der Nadel (18) zu bilden;
**dadurch gekennzeichnet**, daß
f) ein Spritzenhalter (14) zum Halten der Spritze (12) vorgesehen ist,
g) wobei der Spritzenhalter (14) eine Lippe (24) und eine Oberfläche (25) aufweist, die mit einer Nadelanlagefläche (16) zwischen der Spritze (12) und der Nadel (18) in Kontakt kommt, so daß die Spritze (12) und der Spritzenhalter (14) untereinander drehbar angeordnet sind, während eine Längsbewegung der Spritze (12) bezüglich des Spritzenhalters (14) behindert ist;
h) wobei der Nadelschutz (26) mit dem Spritzenhalter (14) einstückig ausgebildet ist,
i) wobei die Spritze (12) für einen automatischen Einsatz angepaßt ist,
j) wobei das Rohrnetz (41) eine einer falschen Behandlung widerstehende Scheibe (46) mit einer ringförmigen Anlagefläche (45) aufweist, die als Handgriff dient, wobei die Scheibe (46) mit dem Gehäuse einer Spritzenpumpe eine mechanische Störeinrichtung bildet, die damit zusammenwirkt, um ein Falschbehandeln oder ein Lösen zu verhindern;
k) wobei die Einstichstelleneinrichtung (48) vom Rohrset (41) entfernbar ist, um einen weiblichen Verschluß-Verbinder offenzulegen.

2. Verabreichungssystem nach Anspruch 1, **dadurch gekennzeichnet**, daß das Rohrset (41) eine nicht hebernde (anti-siphoning) Einrichtung (42) zwischen der Einstichstelleneinrichtung (48) und dem Schlauch (50) aufweist.

3. Verabreichungssystem nach Anspruch 2, **dadurch gekennzeichnet**, daß die Einstichstelleneinrichtung (48) von der nicht hebernden (anti-siphoning) Einrichtung (42) entfernbar ist, um einen weiblichen Luer-lock-Verbinder (56) offenzulegen.

4. Verabreichungssystem nach Anspruch 3, **dadurch gekennzeichnet**, daß der Schlauch (50) von der nicht hebernden (anti-siphoning) Einrichtung (42) zu einem männlichen Luer-lock-Verbinder führt.

5. Verabreichungssystem nach Anspruch 1, **dadurch gekennzeichnet**, daß der Nadelschutz (26) ein hohler Zylinder ist, wobei die Nadel (18) sich entlang eines Abschnittes einer Längsachse des Nadelschutzes (26) erstreckt.

6. Verabreichungssystem nach Anspruch 5, **dadurch gekennzeichnet**, daß eine Nadelhülse (28) vorgesehen ist, die über der Nadel (18) entfernbar angeordnet ist.

## Revendications

1. Dispositif de distribution de médicaments comportants:
a) une seringue (12) munie d'une aiguille (18),
b) un piston (36) en prise dans ladite seringue (12),
c) l'aiguille ayant une extrémité s'étendant à partir de ladite seringue et comportant un passage pour l'écoulement de liquide provenant de ladite seringue à travers ladite aiguille en réponse à une poussée dudit piston longitudinalement à travers ladite seringue;
d) une protection (26) d'aiguille s'étendant au-delà de l'extrémité de l'aiguille (18),
e) un ensemble (41) formant tube muni de moyens (48) formant orifice de ponction, dans lequel le tube de l'ensemble (41) formant tube s'étend depuis les moyens (48) formant orifice de ponction et dans lequel les moyens (48) formant orifice de ponction ont un diamètre égal au diamètre intérieur de la protection (26) d'aiguille, de telle sorte que lorsque les moyens (48) formant orifice de ponction sont insérés dans la protection (26) d'aiguille, l'aiguille (18) est positionnée de manière précise contre les moyens (48) formant orifice de ponction et pour ainsi former une liaison étanche au liquide entre les moyens (48) formant orifice de ponction et l'aiguille (18),
caractérisé en ce que :
f) un dispositif (14) de maintien de seringue est agencé pour maintenir la seringue (12),
g) le dispositif (14) de maintien de seringue comporte une lèvre (24) et une surface (25) qui viennent en contact avec un moyeu (16) d'aiguille situé entre la seringue (12) et l'aiguille (18) de manière telle que la seringue (12) et le dispositif (14) de maintien de seringue peuvent tourner l'un autour de l'autre alors qu'un mouvement longitudinal de la seringue (12) par rapport au dispositif (14) de maintien de seringue est empêché;
h) la protection (26) d'aiguille est venue de matière avec le dispositif (14) de maintien de seringue,
i) la seringue (12) est adaptée pour être entrainée de manière automatique,
j) l'ensemble (41) formant tube comporte un disque (46) anti-mauvaise utilisation ayant un moyeu annulaire (45) servant en tant que saisie manuelle, dans lequel le disque (46) forme une interférence mécanique avec le boîtier d'une pompe de seringue, en coopérant pour empêcher une mauvaise utilisation ou une déconnexion,
k) les moyens (48) formant orifice de ponction peuvent être enlevés de l'ensemble (41) formant tube afin d'exposer un raccord femelle formant verrou.

2. Dispositif selon la revendication 1, caractérisé en ce que l'ensemble (41) formant tube comporte des moyens (42) anti-siphonnement situés entre les moyens (48) formant orifice de ponction et le tube (50).

3. Dispositif selon la revendication 2, caractérisé en ce que les moyens (48) formant orifice de ponction peuvent être enlevés des moyens (42) anti-siphonnement afin d'exposer un raccord femelle (56) de Luer formant verrou.

4. Dispositif selon la revendication 3, caractérisé en ce que le tube (50) part des moyens (42) anti-siphonnement vers un raccord mâle (52) de Luer formant verrou.

5. Dispositif selon la revendication 1, caractérisé en ce que la protection (26) d'aiguille est un cylindre creux, l'aiguille (18) s'étendant le long d'une partie de l'axe longitudinal de la protection (26) d'aiguille.

6. Dispositif selon la revendication 5, caractérisé en ce qu'une gaine (28) d'aiguille est agencée qui s'adapte de manière amovible sur l'aiguille (18).
